(19) Europäisches Patentamt
European Patent Office
Office européen des brevets

(11) **EP 2 199 254 A1**

(12) **EUROPEAN PATENT APPLICATION**

(43) Date of publication:
**23.06.2010 Bulletin 2010/25**

(21) Application number: **08253980.0**

(22) Date of filing: **11.12.2008**

(51) Int Cl.:
*C01B 3/02* (2006.01)    *C01B 3/32* (2006.01)
*C01B 3/48* (2006.01)    *C01B 3/50* (2006.01)
*C01B 3/58* (2006.01)    *C01C 1/04* (2006.01)
*C10G 2/00* (2006.01)    *C07C 29/151* (2006.01)

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **BP p.l.c.**
**London SW1Y 4PD (GB)**

(72) Inventor: **The designation of the inventor has not
yet been filed**

(74) Representative: **De Kezel, Eric et al**
**BP International Limited**
**Global Patents & Technology Law**
**Chertsey Road**
**Sunbury-on-Thames, Middlesex**
**TW16 7LN (GB)**

(54) **Integrated gas refinery**

(57)    The present invention relates to an integrated synthesis gas refinery plant and a process for the simultaneous production from a single synthesis gas stream X of a hydrogen stream useful for the production of ammonia, a hydrogen rich synthesis gas stream useful for the production of methanol, and a hydrogen depleted synthesis gas stream useful for the production of hydrocarbons like gasoline and/or diesel.

**Description**

The present invention relates to a process for the simultaneous production of a hydrogen stream A useful for the production of product A, a hydrogen rich synthesis gas stream B useful for the production of product B, and a hydrogen depleted synthesis gas stream C useful for the production of product C from a single synthesis gas stream X.

In particular, the present invention relates to an integrated synthesis gas refinery plant and a process for the simultaneous production from a single synthesis gas stream X of a hydrogen stream useful for the production of ammonia, a hydrogen rich synthesis gas stream useful for the production of methanol, and a hydrogen depleted synthesis gas stream useful for the production of hydrocarbons like naphtha and diesel.

BRIEF DESCRIPTIONS OF DRAWING

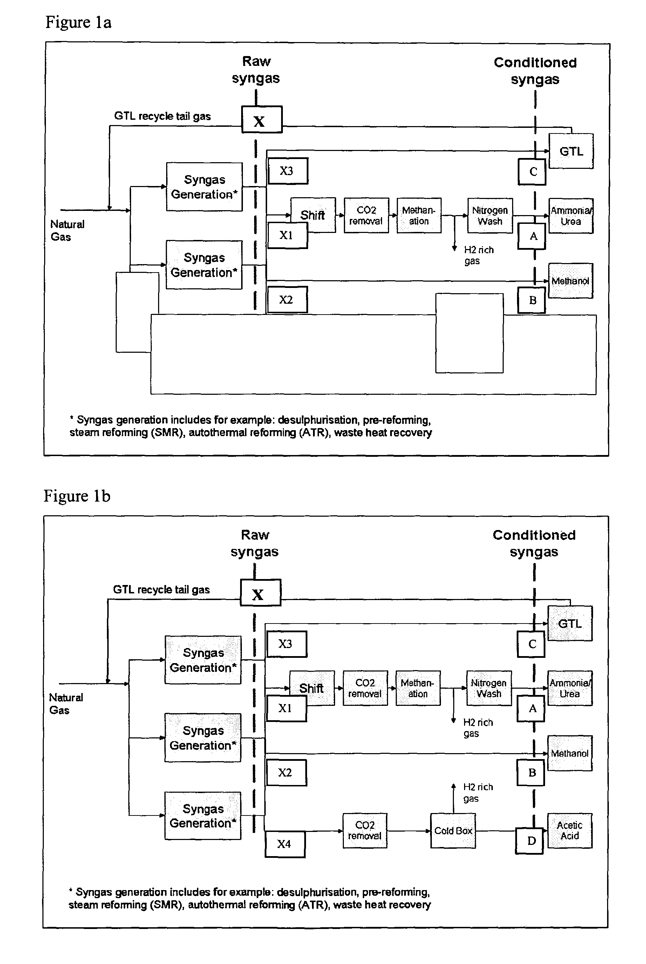

Figure 1a represents one specific embodiment according to the present invention wherein three products are produced from synthesis gas. In Figure 1a, the process according to the present invention is schematically depicted as follows:

- a source of natural gas is introduced into multiple synthesis gas generation reactors to generate the single synthesis gas source, hereinafter referred as the "hydrogen depleted synthesis gas", used in the downstream operations,
- said generated synthesis gas is divided into three fractions,
- a first fraction of the synthesis gas is used as the synthesis gas source of the gas-to-liquids plant,
- a second fraction of the synthesis gas is subjected to a water gas shift reaction step followed by a CO2 separation, a methanation step and a nitrogen wash step; the hydrogen produced during this treatment is used as the hydrogen source of the ammonia plant,
- a third fraction of the synthesis gas is enriched with hydrogen coming from the above second fraction treatment and the resulting enriched hydrogen synthesis gas, hereinafter called the "hydrogen rich synthesis gas", is used as the source of synthesis gas of the methanol plant.

Figure 1b represents another specific embodiment according to the present invention wherein four products are produced from synthesis gas. In Figure 1b, the process according to the present invention is schematically depicted as follows:

- a source of natural gas is introduced into multiple synthesis gas generation reactors to generate the single synthesis gas source, hereinafter referred as the "hydrogen depleted synthesis gas", used in the downstream operations,
- said generated synthesis gas is divided into four fractions,
- a first fraction of the synthesis gas is used as the synthesis gas source of the gas-to-liquids plant,
- a second fraction of the synthesis gas is subjected to a water gas shift reaction step followed by a CO2 separation, a methanation step and a nitrogen wash step; the hydrogen produced during this treatment is used as the hydrogen source of the ammonia plant,
- a third fraction of the synthesis gas is enriched with hydrogen coming from the above second fraction treatment and the resulting enriched hydrogen synthesis gas, hereinafter called the "hydrogen rich synthesis gas", is used as the source of synthesis gas of the methanol plant,
- a fourth fraction of the synthesis gas is subjected to an absorber which removes carbon dioxide and a low temperature separator to obtain a carbon monoxide stream, which is used as the source of carbon monoxide of the acetic acid plant.

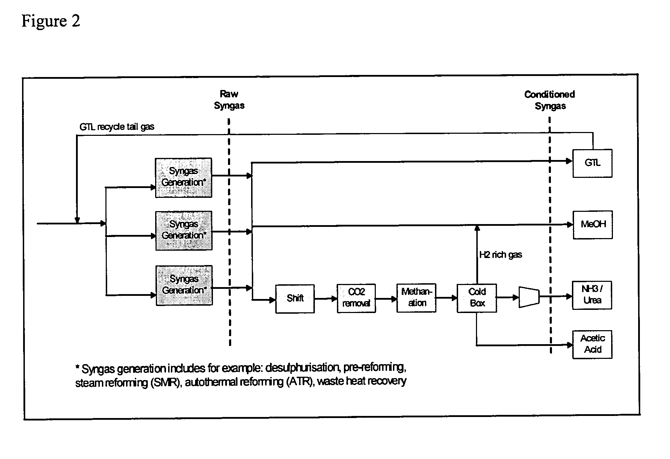

Figure 2 represents another embodiment according to the present invention wherein four products are produced from synthesis gas. In Figure 2, the process according to the present invention is schematically depicted as follows:

- a source of natural gas is introduced into multiple synthesis gas generation reactors to generate the single synthesis gas source, hereinafter referred as the "hydrogen depleted synthesis gas", used in the downstream operations,
- said generated synthesis gas is separated into 3 fractions,
- a first fraction of the synthesis gas is used as the synthesis gas source of the gas-to-liquids plant,
- a second fraction of the synthesis gas is subjected to a water gas shift reaction step followed by a CO2 separation, a methanation step and a low temperature separation step; the hydrogen produced during this treatment is used as the hydrogen source of the ammonia plant; the carbon monoxide produced during this treatment is used as the carbon monoxide source of the acetic acid plant,
- a third fraction of the synthesis gas is enriched with hydrogen coming from the above second fraction treatment and the resulting enriched hydrogen synthesis gas, hereinafter called the "hydrogen rich synthesis gas", is used as the source of synthesis gas of the methanol plant.

DETAILED DESCRIPTION

Thus, the present invention provides a process for the simultaneous production of a hydrogen stream A useful for the production of product A; a hydrogen rich synthesis gas stream B useful for the production of product B; a hydrogen depleted synthesis gas stream C useful for the production of product C; from a single synthesis gas stream X **characterised in that**:

> a) the single synthesis gas stream X has a synthesis gas molar ratio calculated as $H_2/CO$ optimized for the production of product C,
> b) the single synthesis gas stream X is separated into a synthesis gas stream X1, a synthesis gas stream X2 and a synthesis gas stream X3,
> c) the synthesis gas stream X1 is subjected to a water gas shift reaction step to convert the CO from the synthesis gas stream X1 into $CO_2$ and $H_2$,
> d) the $CO_2$ and $H_2$ from step c) are respectively separated and recovered,
> e) a fraction of the $H_2$ from step d) is used as the hydrogen stream A,
> f) a fraction of the $H_2$ from step d) is combined with synthesis gas stream X2 which is then used as the hydrogen rich synthesis gas stream B, and
> g) the synthesis gas stream X3 is used as the hydrogen depleted synthesis gas stream C.

As indicated hereinabove, a characterizing feature according to the present invention is that the synthesis gas stream X has a synthesis gas molar ratio calculated as $H_2/CO$ optimized for the production of product C. Therefore, it is clear that the present invention requires in all circumstances that the synthesis gas molar ratio required for the production of chemical C is lower than the synthesis gas molar ratio required for the production of chemical B which is also lower than the synthesis gas molar ratio required for the production of chemical A.

The Applicants have indeed unexpectedly found that there is great benefit for configuring the reforming section to meet within acceptable limits the lowest synthesis gas molar ratio ($H_2/CO$) requirement of the downstream plants. Synthesis gas conditioning for downstream plants requiring higher $H_2/CO$ ratio can then be conducted separately, for example by the transformation of carbon monoxide to hydrogen via the water gas shift reaction. The Applicants have found that this was highly beneficial as generating hydrogen-rich synthesis gas stream in a separate water shift reaction (as opposed to generating it directly in a steam methane reformer for example) results in lower overall carbon dioxide ($CO_2$) emissions as explained hereinafter. In addition, this configuration shifted atmospheric $CO_2$ make to high-pressure process $CO_2$ make, which further reduces $CO_2$ emissions when high pressure $CO_2$ is captured and sequestrated, as explained hereinafter.

According to a preferred embodiment of the present invention, the single synthesis gas stream X has a synthesis gas molar ratio calculated as $H_2/CO$ of from 1.6 to 2.5 and preferably from 1.7 to 2.2.

SYNTHESIS GAS

According to the present invention, the single synthesis gas stream X can come from any appropriate hydrocarbon feedstock. Said hydrocarbon feedstock used for synthesis gas generation is preferably a carbonaceous material, for example biomass, plastic, naphtha, refinery bottoms, crude synthesis gas (from underground coal gasification or biomass gasification), smelter off gas, municipal waste, coal, and/or natural gas, with coal and natural gas being the preferred sources, and natural gas being the most preferable source.

Natural gas commonly contains a range of hydrocarbons (e.g. C1-C3 alkanes), in which methane predominates. In addition to this, natural gas will usually contain nitrogen, carbon dioxide and sulphur compounds. Preferably the nitrogen content of the feedstock is less than 40 wt %, more preferably less than 10 wt % and most preferably less than 1 wt %. According to a preferred embodiment of the present invention the hydrocarbon feedstock may either comprise a single feedstock, or a plurality of independent feedstocks.

According to the present invention, the said hydrocarbon feedstock(s) is first fed into a least one synthesis gas generator(s), having an external heat input, in order to produce a stream comprising essentially carbon oxide(s) and hydrogen (commonly known as synthesis gas) as well as water, traces of unconverted hydrocarbons, nitrogen and inert gas.

Suitable "synthesis gas generation methods" include, but are not limited to, steam reforming (SR), compact reforming (CR), partial oxidation of hydrocarbons (POX), advanced gas heated reforming (AGHR), microchannel reforming, plasma reforming, autothermal reforming (ATR) and all combinations thereof (regardless of whether the said synthesis gas generation methods are operated in series or in parallel).

Synthesis gas generation methods used for producing mixtures of carbon oxide(s) and hydrogen (synthesis gas), in one or more synthesis gas generator(s), are well known. Each of the aforementioned methods has its advantages and disadvantages, and in practice the choice of using a one particular reforming process over another is dictated by economic and available feed stream considerations, as well as obtaining the desired molar ratio of H2/CO in the feedstock resulting from the reforming reaction. A discussion of the available synthesis gas production technologies is provided in both "Hydrocarbon Processing" V78, N.4, 87-90, 92-93 (April 1999) and "Petrole et Techniques", N. 415, 86-93 (July-August 1998).

Processes for obtaining the synthesis gas by catalytic partial oxidation of hydrocarbons (as mentioned above) in a microstructured reactor are exemplified in "IMRET 3: Proceedings of the Third International Conference on Microreaction Technology", Editor W Ehrfeld, Springer Verlag, 1999, pages 187-196. Alternatively, the synthesis gas may be obtained by short contact time catalytic partial oxidation of hydrocarbonaceous feedstocks as described in EP 0303438.

The synthesis gas can also be obtained via a "Compact Reformer" process as described in "Hydrocarbon Engineering", 2000, 5, (5), 67-69; "Hydrocarbon Processing", 79/9, 34 (September 2000); "Today's Refinery", 15/8, 9 (August 2000); WO 99/02254; and WO 200023689.

According to an embodiment of the present invention, the synthesis gas is generated via at least one steam reforming apparatus (e.g. a steam methane reformer). The steam reforming apparatus configuration is preferably used together with at least one other suitable synthesis gas generator (e.g. an auto-thermal reformer or a partial oxidation apparatus), wherein the said generators are preferably connected in series.

The steam reforming reaction is highly endothermic in nature. Hence, the reaction is commonly catalysed within the tubes of a reformer furnace. When natural gas is chosen as the hydrocarbon feedstock, the endothermic reaction heat that is needed is supplied by burning a fuel (e.g. additional amounts of natural gas or hydrogen). Simultaneous to the steam reforming reaction, the water/gas shift reaction also takes place within the reactor. Since sulphur is a known poison towards the typical catalysts required for the reaction within the steam reformer, the chosen hydrocarbon feedstock is preferably de-sulphurised prior to entering the said reformer.

Additionally, the steam reformer requires a high steam to carbon ratio to prevent carbon from being deposited on the catalyst, and also to ensure high conversion to carbon monoxide, and so the preferred molar ratio of steam to carbon (i.e. the carbon that is present as hydrocarbons) is between 1 and 3.5, preferably between 1.2 and 3.

According to another embodiment of the present invention the steam reforming apparatus employed is a compact reformer. The said compact reformer integrates preheating, steam reforming and waste process heat recovery in a single process compact unit. The reformer design typically resembles a conventional shell-and-tube heat exchanger that is compact when compared to, for example, a conventional steam methane reformer design configuration. The steam reforming reactions occur within the tubes of the said reactor, which are filled with conventional catalyst. Heat for the endothermic steam reforming reaction is provided on the shellside, where the tubes are heated by combustion of a fuel/air mixture in amongst flames. Heat transfer occurs more efficiently in what is described as a highly countercurrent device. Preferably, the shell side combustion zone also is at elevated pressure. As such elevated pressure is believed to contribute to a more-efficient convective heat transfer to the tubes.

Typically, for commercial synthesis gas production, the pressure at which the synthesis gas is produced ranges from approximately 1 to 100 BAR and preferably from 15 to 55 BAR; and the temperatures at which the synthesis gas exits the final reformer ranges from approximately 650°C to 1100°C Since, the high temperatures are necessary in order to produce a favourable equilibrium for synthesis gas production, and to avoid metallurgy problems associated with carbon dusting.

According to a preferred embodiment of the present invention, during synthesis gas generation, an additional stage may be employed whereby the feedstock is first purified to remove sulphur and other potential catalyst poisons (such as halides or metals e.g. Hg) prior to being converted into synthesis gas; alternatively this treatment can also be performed after synthesis gas preparation for example, when coal or biomass are used.

As indicated hereinabove, the present invention provides a process for the simultaneous production of a hydrogen stream A useful for the production of product A; a hydrogen rich synthesis gas stream B useful for the production of product B; a hydrogen depleted synthesis gas stream C useful for the production of product C; from a single synthesis gas stream X **characterised in that**:

a) the single synthesis gas stream X has a synthesis gas molar ratio calculated as H2/CO optimized for the production of product C,
b) the single synthesis gas stream X is separated into a synthesis gas stream X1, a synthesis gas stream X2 and a synthesis gas stream X3,
c) the synthesis gas stream X1 is subjected to a water gas shift reaction step to convert the CO from the synthesis gas stream X1 into CO2 and H2,
d) the CO2 and H2 from step c) are respectively separated and recovered,
e) a fraction of the H2 from step d) is used as the hydrogen stream A,
f) a fraction of the H2 from step d) is combined with synthesis gas stream X2 which is then used as the hydrogen rich synthesis gas stream B, and

g) the synthesis gas stream X3 is used as the hydrogen depleted synthesis gas stream C.

According to a preferred embodiment of the present invention, product A is ammonia; product B is methanol; product C is a hydrocarbon mixture comprising naphtha and diesel.
For producing ammonia as product A, nitrogen is combined with the hydrogen stream A of hereinabove step e).
The present invention also provides a process for the simultaneous production of a hydrogen stream A useful for the production of product A; a hydrogen rich synthesis gas stream B useful for the production of product B; a hydrogen depleted synthesis gas stream C useful for the production of product C; and a carbon monoxide stream D, useful for the production of product D; from a single synthesis gas stream X **characterised in that**:

a) the single synthesis gas stream X has a synthesis gas molar ratio calculated as H2/CO optimized for the production of product C,
b) the single synthesis gas stream X is separated into a synthesis gas stream X1, a synthesis gas stream X2, a synthesis gas stream X3 and a synthesis gas stream X4,
c) the synthesis gas stream X1 is subjected to a water gas shift reaction step to convert the CO from the synthesis gas stream X1 into CO2 and H2,
d) the CO2 and H2 from step c) are respectively separated and recovered,
e) a fraction of H2 recovered from step d) is used as the hydrogen stream A,
f) a fraction of the H2 from step d) is combined with synthesis gas stream X2 which is then used as the hydrogen rich synthesis gas stream B,
g) the synthesis gas stream X3 is used as the hydrogen depleted synthesis gas stream C, and
h) the synthesis gas stream X4 is treated to remove the carbon dioxide and hydrogen thereof; and the resulting carbon monoxide stream is used as a carbon monoxide source of stream D.

According to a preferred embodiment of the present invention, product A is ammonia; product B is methanol; product C is a hydrocarbon mixture comprising naphtha and diesel; and product D is acetic acid. For producing ammonia as product A, nitrogen is combined with the hydrogen stream A of hereinabove step e).
According to an embodiment of the present invention, the hydrogen recovered from hereinabove step h) can also advantageously be used as either a fraction of the source of hydrogen for the hydrogen stream A and/or as a fraction of the source of hydrogen for the hydrogen rich synthesis gas stream B.
According to an additional embodiment of the present invention, a fraction of the hydrogen stream A and/or a fraction of the hydrogen recovered from hereinabove step h) can also advantageously be exported for sale.
As indicated hereinabove, a part of the synthesis gas stream (X1) is subjected to a water gas shift reaction step to convert the CO from the said synthesis gas stream (X1) into CO2 and H2. This water gas shift reaction step consists of adding steam to the synthesis gas stream (X1) and subjecting the resulting mixture to a water gas-shift reaction step, in order to convert a majority of the CO present, into CO2 and H2, this step usually leaves some residual amount of CO in the synthesis gas stream, typically 0.3% by volume. This is followed by CO2 removal to obtain a hydrogen stream with a much reduced carbon dioxide content. It is known that the remaining oxygen bearing compounds (CO and CO2) are poisonous towards ammonia synthesis catalyst; said poisons are thus preferably removed from the hydrogen stream, e.g. by using a methanator. Said methanator would then convert these residual carbon oxides to methane and water. The methanated synthesis gas (hydrogen) stream is then preferably cooled and transferred to a nitrogen wash system where methane is separated. Said nitrogen preferably comes from an air separation unit and it is added to the hydrogen stream at the right stoichiometric ratio for the ammonia synthesis unit, i.e. a molar ratio of H2/N2 of about 2.5 to 3.5. The recovered CO2 can be sequestrated. The recovered H2 can then be used as feedstock to the ammonia plant and also as a feedstock to the methanol plant.
As indicated above, the water gas shift reaction is used to convert carbon monoxide to carbon dioxide and hydrogen through a reaction with steam e.g.

$$CO + H_2O = CO_2 + H_2$$

The reaction is exothermic, which means the equilibrium shifts to the right at lower temperatures conversely at higher temperatures the equilibrium shifts in favour of the reactants. Conventional water gas shift reactors use metallic catalysts in a heterogeneous gas phase reaction with CO and steam. Although the equilibrium favours formation of products at lower temperatures the reaction kinetics are faster at elevated temperatures. For this reason the catalytic water gas shift reaction is initially carried out in a high temperature reactor at 350-370°C and this is followed frequently by a lower temperature reactor typically 200-220°C to improve the degree of conversion (Kirk-Othmer 1995, Ulllman's 1989). The conversions of CO are typically 90% in the first reactor and a further 90% of the remaining CO is converted in the low temperature reactor. Development of higher activity water gas shift catalysts will allow reduction in the reaction temperatures employed. Other non metallic catalysts such as oxides and mixed metal oxides such as Cu/ZnO are known to catalyse this reaction. The degree of conversion of the CO can also be increased by adding more than the stoichiometric amount of steam but this incurs an additional heat penalty. Methane and nitrogen are inert under typical water gas shift conditions.

In ammonia and hydrogen production, carbon dioxide (CO2) is an unavoidable byproduct of the synthesis gas generation step (regardless of whether the route used is natural gas steam reforming, hydrocarbon partial oxidation, or coal gasification), which must be separated before further downstream processing is possible. Virtually all commercial processes for CO2 separation are based on absorption in liquid solvents. The solvents used may be categorized into two types - chemical solvents (such as aqueous solutions of monoethanolamine or potassium carbonate, where the mechanism of absorption is via a reversible chemical reaction) or physical solvents (such as methanol used in "Rectisol" or dimethyl ethers of polyethylene glycols used in "Selexol", where the absorption of CO2 and other acid gases is without chemical reactions). The solvents typically contain an activator to promote mass transfer. It is possible to remove carbon dioxide to less than 1000 ppm in many absorption systems. However, trace amount of carbon oxides always remain and are removed by methanation as mentioned below.

$$CO + 3H_2 \Leftrightarrow CH_4 + H_2O$$

$$CO_2 + 4H_2 \Leftrightarrow CH_4 + 2H_2O$$

Following CO2 removal, the small amount of carbon oxides (e.g. CO, CO2) are converted in the methanator to methane by passing the gas over an iron or nickel catalyst. Carbon oxides are reduced to trace levels because they are ammonia synthesis catalyst poisons. The main methanation reactions are highly exothermic and are favored by low temperatures and high pressures. The reaction rate increases both with increased temperature and pressure. Carbon deposition can occur during methanation. However, with the large excess of hydrogen in synthesis gas, no problems in carbon formation are usually encountered. Very low carbon oxide levels (<10ppm) can be produced by methanation. The disadvantage of methanation is that some hydrogen is consumed and the process can only be used for residual quantities of carbon dioxides, otherwise the inert level of the synthesis gas would be too high. The methanated synthesis gas is then cooled and dried to remove traces of water over alumina or molecular sieves before entering the nitrogen wash stage.

The stoichiometry for ammonia synthesis from hydrogen and nitrogen are:

$$3 H_2 + N_2 \Leftrightarrow 2 NH_3$$

In addition, since the synthesis reaction is also equilibrium controlled and conversion per pass is low, the synthesis loop requires a large recycle. Inert impurities therefore lower the efficiency since large purge stream must be removed to avoid accumulation of impurities in the recycle loop. Cryogenic washing with liquid nitrogen is used to remove methane and argon to very low levels.

Following any necessary purification, the hydrogen stream is compressed and finally reaches the ammonia converter where the contained hydrogen and nitrogen chemically combine over a catalyst to produce ammonia. All commercial processes for the manufacture of ammonia depend on the equilibrium reaction between hydrogen and nitrogen as shown in the reaction above. This equilibrium reaction is favored by increased pressure and decreased temperatures. At a given temperature and pressure, the equilibrium ammonia concentration decreases linearly with an increasing concentration of inerts. Equilibrium is also affected by the hydrogen-nitrogen ratio.

Whilst the equilibrium indicates that conversion increases continuously with pressure, the optimum synthesis pressure in current ammonia plant design is within the range of 150-375 atm. The catalyst is commonly iron, which may be promoted with aluminum, potassium and/or calcium. A wide variety of ammonia synthesis designs are available and they are described in the literature.

Conversion efficiency (i.e. the ratio of actual ammonia in the gas to that theoretically possible under the operating conditions) always increases with increasing temperature. However, above 480-550°C, iron catalysts begin to deteriorate and some means must be used to cool or prevent overheating. Depending to some degree on the catalyst, the normal catalyst inlet temperature in most commercial converters is about 400°C and the maximum hot spot temperature is not allowed above 525°C. The composition of the hydrogen/nitrogen stream plays an important part in determining the conversion. Both conversion and conversion efficiency are functions of the ratio of hydrogen to nitrogen. Both the equilibrium ammonia conversion and rate of conversion increase with pressure. However, conversion efficiency has been found to decrease some 15-20% when pressure was increased from 151 to 317 atm.

The synthesis of methanol requires a typical composition of the synthesis gas with a stoichiometric number of between 2.0 to .2.15 ("Sn") and is preferably 2.08, a carbon dioxide concentration typically in the range between 2 and 8% by volume and a nitrogen concentration typically of less than 0.5% by volume. The stoichiometric number (Sn) is calculated according to the following formula:

$$Sn = \frac{([H2] - ([CO2]))}{([CO2] + ([CO]))}$$

In this formula, [H2], [CO2] and [CO] represent the molar concentrations of hydrogen, carbon dioxide and carbon monoxide in the synthesis gas.

According to the present invention, a fraction of the H2 separated and recovered from the treatment of the synthesis gas stream X1 is combined with synthesis gas stream X2 which is then used as methanol synthesis gas. Preferably, according to an embodiment of the present invention, said resulting hydrogen rich synthesis gas stream is then introduced into a methanol synthesis unit, in order to produce a stream comprising methanol. Preferably the Sn molar ratio, (H2-CO2):(CO+CO2), of said synthesis gas feed stream fed into the methanol synthesis unit is greater than 1.6, more preferably greater than 1.8 and most preferably greater than 2.0. Preferably the Sn molar ratio, (H2-CO2): (CO+CO2), of said synthesis gas feed stream fed into the methanol synthesis unit is less than 3.0, more preferably less than 2.5 and most preferably less than 2.2.

According to a preferred embodiment of the present invention, the methanol synthesis unit may be any unit that is suitable for producing methanol, for example a fixed bed reactor, which can be run with or without external heat exchange equipments e.g. a multi-tubular reactor; or a fluidised bed reactor; or a void reactor.

Preferably the methanol synthesis unit is operated at a temperature of greater than 200°C, more preferably greater than 220°C and most preferably greater than 240°C; and preferably less than 310°C, more preferably less than 300 °C and most preferably less than 290°C. Preferably the methanol synthesis unit is operated at pressure of greater than 2 MPa and most preferably greater than 5 MPa; and preferably less than 10 MPa and most preferably less than 9MPa. In fact, since methanol synthesis is an exothermic reaction, the chosen temperature of operation is governed by a balance of promoting the forward reaction (i.e. by not adversely affecting the equilibrium) and aiding the rate of conversion (i.e. higher productivity).

The catalysts used for methanol synthesis can be divided into 2 groups:

   i. the high pressure zinc catalysts, composed of zinc oxide and a promoter; and
   ii. low pressure copper catalysts, composed of zinc oxide, copper oxide and a promoter.

Hence, according to a preferred embodiment of the present invention, the preferred methanol synthesis catalyst is a mixture of copper, zinc oxide, and a promoter such as, chromia or alumina. Under the aforementioned operating conditions, these said mixtures can catalyse the production of methanol from carbon monoxide and hydrogen with a high selectivity.

Production of liquid fuels using Fischer Tropsch synthesis consists of three discrete steps. In the first step the hydrocarbon feed (e.g. natural gas, coal, biomass or waste) is converted to synthesis gas. This synthesis gas is then fed to a second stage to be converted to paraffinic wax and light hydrocarbons via the fischer tropsch synthesis. The liquid streams are then passed to a third step, where they are hydrocracked into the desired range and distilled to produce the final products.

The following is the general Fischer-Tropsch synthesis reaction:

$$[CO + 2H_2]_n + H_2 \rightarrow CH_3(CH_2)_{n-2}CH_3 + nH_2O$$

$$CO + 3H_2 \rightarrow CH_4 + H_2O$$

$$CO + H_2O \rightarrow CO_2 + H_2$$

Unwanted side reactions result in the formation of methane and carbon dioxide. There are reaction paths other than the straightforward chain addition. Olefins, alcohols and short chain aldehydes can also be formed.

The synthesis of FT products requires a typical composition of the synthesis gas with a H2:CO ratio of 1.6 to 2.5. The Fischer-Tropsch synthesis reactor entails the conversion of synthesis gas with cobalt or iron based catalyst to produce paraffinic hydrocarbons (wax and light hydrocarbon) and one equivalent of water per carbon atom of product. The reaction is highly exothermic, and poor temperature control can lower the selectivity to higher paraffins.

In the last overall step long chain molecules in the wax and hydrocarbon liquids are isomerised and cracked into shorter molecules on a commercial hydro cracking catalyst. The reaction consists of two steps, cracking of large wax molecules into chains of approximately similar length, and their isomerisation into methyl isomers. The reaction rate for cracking depends on the chain length, so shorter chain straight run product is relatively unaffected by passing through the hydrocracker. Oxygenate compounds however react to form paraffins and water.

The stream from the hydrocracker is separated in the Fractionator into the final hydrocarbon products, e.g. diesel and naptha, with the unconverted wax being recycled to the hydrocracker.

**Claims**

1. A process for the simultaneous production of a hydrogen stream A useful for the production of product A; a hydrogen rich synthesis gas stream B useful for the production of product B; a hydrogen depleted synthesis gas stream C useful for the production of product C; from a single synthesis gas stream X **characterised in that**:

    a) the single synthesis gas stream X has a synthesis gas molar ratio calculated as H2/CO optimized for the production of product C,
    b) the single synthesis gas stream X is separated into a synthesis gas stream X1, a synthesis gas stream X2 and a synthesis gas stream X3,
    c) the synthesis gas stream X1 is subjected to a water gas shift reaction step to convert the CO from the synthesis gas stream X1 into CO2 and H2,
    d) the CO2 and H2 from step c) are respectively separated and recovered,
    e) a fraction of the H2 from step d) is used as the hydrogen stream A,
    f) a fraction of the H2 from step d) is combined with synthesis gas stream X2 which is then used as the hydrogen rich synthesis gas stream B, and
    g) the synthesis gas stream X3 is used as the hydrogen depleted synthesis gas stream C.

2. A process according to claim 1, wherein product A is ammonia; product B is methanol; product C is a hydrocarbon mixture comprising naphtha and diesel.

3. A process for the simultaneous production of a hydrogen stream A, useful for the production of product A; a hydrogen rich synthesis gas stream B, useful for the production of product B; a hydrogen depleted synthesis gas stream C, useful for the production of product C; and a carbon monoxide stream D, useful for the production of product D; from a single synthesis gas stream X **characterised in that**:

    a) the single synthesis gas stream X has a synthesis gas molar ratio calculated as H2/CO optimized for the production of product C,
    b) the single synthesis gas stream X is separated into a synthesis gas stream X1, a synthesis gas stream X2, a synthesis gas stream X3 and a synthesis gas stream X4,
    c) the synthesis gas stream X1 is subjected to a water gas shift reaction step to convert the CO from the synthesis gas stream X1 into CO2 and H2,
    d) the CO2 and H2 from step c) are respectively separated and recovered,
    e) a fraction of H2 recovered from step d) is used as the hydrogen stream A,
    f) a fraction of the H2 from step d) is combined with synthesis gas stream X2 which is then used as the hydrogen rich synthesis gas stream B,
    g) the synthesis gas stream X3 is used as the hydrogen depleted synthesis gas stream C, and
    h) the synthesis gas stream X4 is treated to remove the carbon dioxide and hydrogen thereof; and the resulting carbon monoxide stream is used as a carbon monoxide source of stream D.

4. A process according to claim 3 wherein product A is ammonia; product B is methanol; product C is a hydrocarbon mixture, comprising naphtha and diesel; and product D is acetic acid.

5. A process according to either of claims 3 or 4, wherein the hydrogen recovered from step h) of claim 3 can also be advantageously used as either a fraction of the source of hydrogen for the hydrogen stream A and/or as a fraction of the source of hydrogen for the hydrogen rich synthesis gas stream B.

6. A process according to any of the preceding claims, wherein the single synthesis gas stream X has a synthesis gas molar ratio calculated as H2/CO of from 1.6 to 2.5 and preferably from 1.7 to 2.2.

7. A process according to any of the preceding claims, wherein the Sn molar ratio, (H2-CO2):(CO+CO2), of the hydrogen rich synthesis gas stream is greater than 1.6, preferably greater than 1.8 and most preferably greater than 2.0; and is less than 3.0, preferably less than 2.5 and most preferably less than 2.2.

Figure 1a

Figure 1b

Figure 2

Raw Syngas

Conditioned Syngas

GTL recycle tail gas

Syngas Generation*

Syngas Generation*

Syngas Generation*

Shift

CO2 removal

Methan-ation

Cold Box

H2 rich gas

GTL

MeOH

NH3 / Urea

Acetic Acid

* Syngas generation includes for example: desulphurisation, pre-reforming, steam reforming (SMR), autothermal reforming (ATR), waste heat recovery

Europäisches
Patentamt
European
Patent Office
Office européen
des brevets

## EUROPEAN SEARCH REPORT

Application Number

EP 08 25 3980

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 342 610 A (AIR PROD & CHEM [US]) 23 November 1989 (1989-11-23) * abstract * * figure 1 * * claims 1,5,6 * ----- | 1-7 | INV. C01B3/02 C01B3/32 C01B3/48 C01B3/50 C01B3/58 C01C1/04 C10G2/00 C07C29/151 |
| A | DE 40 41 147 A1 (RUHR OEL GMBH [DE]) 2 July 1992 (1992-07-02) * the whole document * ----- | 1-7 | |
| A | GB 2 084 973 A (TOYO ENGINEERING CORP) 21 April 1982 (1982-04-21) * abstract * * claims 1,2 * ----- | 1-7 | |
| A | WO 03/097523 A (ACETEX CYPRUS LTD [CY]; THIEBAUT DANIEL MARCEL [FR]) 27 November 2003 (2003-11-27) * abstract * * claims 1,15 * * pages 10-17 * * figures 1-4 * ----- | 1-7 | |
| A | WO 2006/117499 A (QUARTEY-PAPAFIO ALEXANDER H [GB]) 9 November 2006 (2006-11-09) * abstract * * claims 1-7 * ----- | 1-7 | TECHNICAL FIELDS SEARCHED (IPC) C01B |

The present search report has been drawn up for all claims

| Place of search | Date of completion of the search | Examiner |
|---|---|---|
| Munich | 7 May 2009 | Alvarez Rodriguez, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 08 25 3980

This annex lists the patent family members relating to the patent documents cited in the above-mentioned European search report.
The members are as contained in the European Patent Office EDP file on
The European Patent Office is in no way liable for these particulars which are merely given for the purpose of information.

07-05-2009

| Patent document cited in search report | | Publication date | Patent family member(s) | | Publication date |
|---|---|---|---|---|---|
| EP 0342610 | A | 23-11-1989 | US | 4886651 A | 12-12-1989 |
| DE 4041147 | A1 | 02-07-1992 | CA | 2058246 A1 | 22-06-1992 |
| GB 2084973 | A | 21-04-1982 | NONE | | |
| WO 03097523 | A | 27-11-2003 | AU | 2003232578 A1 | 02-12-2003 |
| | | | CN | 1656012 A | 17-08-2005 |
| WO 2006117499 | A | 09-11-2006 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0303438 A
- WO 9902254 A
- WO 200023689 A

**Non-patent literature cited in the description**

- *Hydrocarbon Processing,* April 1999, vol. 78 (4), 87-9092-93
- *Petrole et Techniques,* July 1998, 86-93
- IMRET 3: Proceedings of the Third International Conference on Microreaction Technology. Springer Verlag, 1999, 187-196
- *Hydrocarbon Engineering,* 2000, vol. 5 (5), 67-69
- *Hydrocarbon Processing,* September 2000, vol. 79/9, 34
- *Today's Refinery,* August 2000, vol. 15/8, 9